# FASCICULE DE BREVET EUROPEEN

(11) **EP 4 114 580 B1**
(45) Date de publication et mention de la délivrance du brevet: **11.02.2026**
(21) Numéro de dépôt: 21708233.8
(22) Date de dépôt: 02.03.2021
(51) Int. Cl.: B05B 11/00, A61F 9/00, G16H 20/13

(54) **MODULE ÉLECTRONIQUE POUR FLACON D'UN PRODUIT OPHTALMIQUE LIQUIDE À POMPE MÉCANIQUE, ET ENSEMBLE COMPORTANT UN MODULE ET UN FLACON**
ELEKTRONISCHES MODUL FÜR EIN FLÄSCHCHEN MIT EINEM FLÜSSIGEN OPHTHALMISCHEN PRODUKT MIT EINER MECHANISCHEN PUMPE UND ANORDNUNG MIT EINEM MODUL UND EINEM FLÄSCHCHEN
ELECTRONIC MODULE FOR A VIAL CONTAINING A LIQUID OPHTHALMIC PRODUCT WITH A MECHANICAL PUMP AND ASSEMBLY COMPRISING A MODULE AND A VIAL

(30) Priorité: 03.03.2020 FR 2002157
(43) Date de publication de la demande: 11.01.2023
(73) Titulaire: Laboratoires THEA, 63100 Clermont-Ferrand (FR)
(72) Inventeur: MERCIER, Fabrice, 63400 Chamaliers (FR)
(74) Mandataire: Santarelli
(86) Numéro de dépôt international: PCT/EP2021/055162
(87) Numéro de publication internationale: WO 2021/175838

(56) Documents cités:
- EP-A1- 1 216 720
- DE-B3- 102015 004 073
- US-A1- 2016 038 695

## Description

La présente invention concerne le domaine de la distribution des produits ophtalmiques, comprenant des solutions pharmaceutiques et autres préparations ophtalmiques liquides, notamment des collyres.

L'invention se rattache en particulier au domaine des dispositifs utilisés pour délivrer des gouttes de produit ophtalmique à partir d'un réservoir en utilisant une pompe mécanique. La présente invention concerne ainsi les flacons de distribution de liquide, et plus particulièrement des flacons de distribution équipés d'un système de pompe pour amorcer la distribution de ce liquide.

Le principe de tels pulvérisateurs a été décrit dans le document FR-2 739 294, dans une application à la pulvérisation nasale.

Le document WO 2010/139883 déposé par la Demanderesse divulgue l'emploi d'un tel pulvérisateur pour une application ophtalmique, avec une ergonomie adaptée pour cet usage.

En effet, dans ce type de flacon, on utilise de façon connue un mécanisme à pompe que l'on actionne en pressant une partie mobile du dispositif.

Afin de concevoir un flacon à l'ergonomie optimale, qui facilite la prise en main et qui permette d'appliquer sur la partie mobile l'effort nécessaire à l'activation de la pompe, il est connu de l'équiper d'une coiffe pour aider à la manœuvre de la partie mobile et donc de la pompe. Une telle coiffe comporte un corps cylindrique qui vient recouvrir au moins en partie le réservoir du flacon, et qui présente à son sommet une paroi supérieure plane percée en son centre pour permettre le passage de l'embout diffuseur du flacon. Lorsque la coiffe est en place sur le flacon, la paroi supérieure vient prendre appui contre la partie mobile de la pompe. On augmente ainsi l'étendue de la zone sur laquelle on appuie pour actionner la pompe et on facilite son fonctionnement à long terme.

Différentes variantes de coiffes peuvent être utilisées avec le flacon pour faciliter l'actionnement de la pompe, en prenant par exemple la forme telle que revendiquée dans le document WO 2010/139883 déposé par la Demanderesse, qui décrit une coiffe en deux parties adaptées à coulisser l'une vers l'autre parallèlement à l'axe de la pompe et qui présentent chacune une ailette latérale de préhension, qui facilite encore l'actionnement des parties du poussoir.

Dans ce contexte, la Demanderesse a également déposé le document WO 2014/170736, qui propose un flacon de distribution de liquide comportant une coiffe qui permet un marquage simple et efficace visant à prévenir l'utilisateur de la première utilisation du flacon, c'est-à-dire permettant à l'utilisateur de constater si le flacon a déjà été ouvert ou non, afin de faciliter la conservation des flacons selon les préconisations d'usage.

Par ailleurs, l'efficacité de nombreux traitements ophtalmiques dépend de plusieurs paramètres, notamment du respect de la posologie de traitement, et, en particulier pour les flacons à pompe mécanique, de l'orientation correcte du flacon lors de l'instillation du produit ophtalmique liquide.

Afin de contrôler la position d'un flacon de produit ophtalmique, dans le but de garantir une position d'instillation correcte, des guides mécaniques qui imposent une position correcte au flacon par application autour de l'oeil sont connus, par exemple au travers des documents JP2003310710 et JP2008295880. Ces systèmes sont néanmoins peu agréables.

Le document EP2912460 divulgue un flacon comportant un capteur de position qui est employé pour mettre en route un capteur optique de goutte. Lorsque la position du flacon est correcte le dispositif déclenche automatiquement les moyens de contrôle de la délivrance de produit. Ce système est complexe, il ne porte pas sur un flacon à pompe mécanique, et ne permet pas à l'utilisateur de choisir l'instant d'instillation. Outre les problèmes précités, la question du contrôle du respect de la posologie demeure irrésolue dans l'art antérieur. Par ailleurs, ce contrôle strict du respect de la posologie ne s'avère pas nécessaire pour tous les traitements ou pour tous les patients, mais peut a contrario être utile sur une période assez longue lors de laquelle sont successivement utilisés plusieurs flacons d'un produit ophtalmique. Un dispositif selon le préambule de la revendication 1 est également connu du document EP1216720A1.

La présente invention vise ainsi à proposer un dispositif permettant de résoudre tout ou partie des problèmes précités.

A cette fin, il est proposé dans l'invention un module électronique selon la revendication 1 et un ensemble comportant un flacon ainsi que ledit module selon la revendication 10.

Le module électronique est indépendant dudit flacon auquel il peut être rapporté. Par « indépendant », il est entendu que le module peut être rapporté et fixé au flacon. Le cas échéant, le module peut ensuite être détaché du flacon. Il s'agit donc d'un module distinct du flacon. Le module, y compris le cas échéant sa coque ou son boîtier, n'est pas formé intégralement avec une partie du flacon. Lorsqu'il est rapporté et fixé à un flacon adapté, le module est en interaction mécanique avec le flacon, de sorte qu'un actionnement de la pompe du flacon actionne le module comme détaillé ci-après. Le module comporte un actionneur configuré de sorte à être entraîné en mouvement par le mouvement de la partie mobile, ledit module comportant un capteur configuré de sorte à être actionné par le mouvement de l'actionneur.

Un module électronique indépendant, et donc adapté à être rapporté à un flacon de produit ophtalmique, présente de nombreux avantages. Cela permet d'équiper un flacon uniquement lorsque cela est nécessaire. Par exemple, lorsque le patient a des difficultés à suivre la posologie prescrite, ou au début d'un traitement lorsqu'il est nécessaire de s'assurer que la posologie ou d'autres paramètres d'administration du produit sont correctement respectés. Par ailleurs, le module électronique peut être transféré d'un flacon à un autre, afin de suivre le traitement d'un utilisateur sur une longue période (supérieure à la durée de traitement offerte par un flacon). Enfin, cela est économique, en ce que le module peut être proposé pour une large gamme de produits, sans avoir à prévoir des gammes de flacons différentes, par produit et selon qu'une transmission ou une mémorisation des données d'actionnement du flacon est souhaitée ou non.

Le module peut être adapté en particulier à un flacon dans lequel la partie mobile est mobile en translation axiale relativement au corps principal. Le capteur peut être un contacteur, l'actionneur étant guidé en translation selon ladite direction axiale et comportant une première surface d'appui sur la partie mobile et une deuxième surface d'appui sur le contacteur.

L'actionneur peut se présenter sous la forme d'une tige s'étendant selon la direction axiale, la tige comportant une première extrémité formant la première surface d'appui, et une deuxième extrémité, opposée à la première extrémité, formant la deuxième surface d'appui.

Le module électronique peut comporter un ressort tendant à ramener l'actionneur dans une position de repos. Le contacteur est alors avantageusement ouvert lorsque l'actionneur est dans la position de repos.

Un contacteur est un dispositif simple et économique, permettant d'avoir une information fiable sur l'actionnement de la pompe d'un flacon à pompe mécanique. Un contacteur est en outre relativement simple à mettre en oeuvre avec un tel flacon, du fait de l'actionnement axial de la pompe, pouvant être mis à profit pour générer une pression axiale sur le contacteur. Un contacteur présente aussi l'avantage de pouvoir servir d'interrupteur au module, de sorte que le module ne consomme de l'énergie électrique, fournie par une pile ou une batterie, que lorsque le contacteur est fermé.

Le module électronique peut comporter un boîtier formant un volume interne de réception d'au moins une carte électronique, ledit boîtier comportant une base et un couvercle, ladite base comportant une ouverture permettant le passage de l'actionneur. Le module électronique est ainsi correctement protégé de l'environnement extérieur, seul l'actionneur dépassant du boîtier. Le couvercle du boîtier peut, dans certains modes de réalisation, être amovible, mais il sera plus généralement inamovible, par exemple scellé lors du montage du module. Le module électronique est sans entretien. Il peut notamment, dans des modes de réalisation préférés, être équipé d'une pile permettant l'alimentation du module pendant par exemple plusieurs semaines, plusieurs mois, voire plusieurs années.

Le module électronique peut comporter au moins un accéléromètre configuré pour fournir une information d'orientation du module électronique.

Un accéléromètre permet de caractériser l'orientation du flacon équipé du module. Or, l'orientation du flacon lors de la distribution du produit est importante. Notamment, pour un flacon à pompe mécanique à actionnement axial, il est généralement nécessaire que le flacon soit vertical lors de l'instillation de produit dans L'instillation demeure correcte dans une plage d'angle relativement limitée autour de la verticale, tandis qu'une mauvaise inclinaison du flacon peut conduire à une mauvaise instillation (quantité de produit délivrée ou reçue par l'oeil insuffisante, point de chute de la goutte délivrée peu précis, etc.).

L'orientation du flacon lors de la distribution du produit est donc un paramètre important dans l'observance d'un traitement, et cette information peut être employée pour qualifier l'observance, et/ou pour l'apprentissage de l'utilisateur à correctement utiliser le flacon.

Le module électronique peut comporter une mémoire électronique adaptée à stocker des données relatives aux actionnements de l'actionneur, notamment le nombre, la date et l'heure des actionnements.

Les données mesurées comportent ainsi les données de base pour contrôler l'observance du traitement.

Le module électronique peut comporter un port de communication, filaire ou sans fil, adapté au transfert de données vers un équipement informatique externe.

Le module électronique peut notamment être adapté à transférer les données vers l'équipement informatique externe selon un protocole Bluetooth^{™}.

La transmission sans fil des données mesurées et/ou collectées par le module permet d'interfacer le module électronique avec un système externe, qui permet le traitement et la visualisation des données. Il peut s'agir de contrôler la bonne observance du traitement, par le patient ou son médecin, ou d'apprendre à l'utilisateur à réaliser des instillations correctes, et donc efficaces, du produit ophtalmique.

L'invention porte également sur un ensemble comportant un flacon d'un produit ophtalmique liquide et un module électronique tel que précédemment défini. Le flacon comporte un corps principal comportant un réservoir et un fond, une tête de distribution et une pompe mécanique, la tête de distribution étant montée sur le corps principal, la tête de distribution comportant une partie mobile dont un mouvement actionne la pompe mécanique pour prélever le produit ophtalmique liquide présent dans le réservoir. Le module électronique est rapporté et fixé soit au corps principal soit à la tête de distribution, de sorte que le mouvement de la partie mobile relativement au corps principal pour prélever le produit ophtalmique liquide présent dans le réservoir entraine en mouvement l'actionneur du module électronique.

La partie mobile d'un tel flacon peut être mobile en translation axiale relativement au corps principal. Ce flacon peut comporter un dispositif d'assistance à la délivrance, le corps principal comportant une première partie du dispositif d'assistance à la délivrance qui coopère avec le réservoir, la première partie du dispositif d'assistance à la délivrance comportant une première ailette latérale, la tête de distribution comportant une deuxième partie du dispositif d'assistance à la délivrance qui coopère avec la partie mobile, la deuxième partie du dispositif d'assistance à la délivrance comportant une deuxième ailette latérale, la première ailette et la deuxième ailette étant sensiblement en regard l'une de l'autre pour permettre le mouvement axial de la partie mobile lorsqu'une force est exercée entre les deux ailettes tendant à les rapprocher axialement l'une de l'autre.

Dans un tel ensemble, lors du mouvement axial de la partie mobile, l'actionneur peut prendre appui sur, et actionné par, la deuxième partie du dispositif d'assistance à la délivrance.

En particulier, la première ailette latérale peut comporter un trou traversé par l'actionneur du module électronique, ledit actionneur prenant appui lors du mouvement axial de la partie mobile sur une excroissance radiale de la deuxième partie du dispositif d'assistance à la délivrance.

Le module électronique objet de la présente invention peut ainsi former, en association avec un flacon de type préexistant, un flacon de produit ophtalmique permettant la collecte de données relatives à l'observance d'un traitement, voire un flacon connecté. Le module électronique proposé ne nécessite pas le développement d'un nouveau flacon pompe. Il est particulièrement adapté à équiper un flacon à pompe mécanique comportant un dispositif d'assistance à la délivrance, d'un type déjà commercialisé par la Demanderesse.

Un module et un ensemble tels que précédemment décrits peuvent trouver une application dans un dispositif de suivi de l'observance d'un traitement thérapeutique, comportant un module électronique tel que défini ci-dessus du type comportant un port de communication, et un équipement informatique externe adapté à communiquer avec ledit module électronique, le module électronique étant configuré pour transmettre des données relatives à l'actionnement de l'actionneur à l'équipement informatique, l'équipement informatique exécutant un logiciel permettant un suivi temporel des actionnements de l'actionneur du module électronique , qui correspondent à une délivrance du produit ophtalmique.

Dans un tel dispositif, l'équipement informatique exécutant le logiciel permet l'enregistrement d'une posologie, et la fourniture à l'utilisateur d'une comparaison entre la posologie enregistrée et les données relatives à l'actionnement de l'actionneur du module électronique transmises à l'équipement informatique.

Il est ainsi proposé une solution complète permettant de comparer le traitement réellement suivi par le patient à la posologie qui lui a été prescrite.

Dans un tel dispositif de suivi de l'observance d'un traitement thérapeutique, le module électronique étant du type défini ci-dessus comportant un accéléromètre, le module peut être configuré pour transmettre des données relatives à son orientation, et l'équipement informatique permet à l'utilisateur de visualiser une information quant à l'orientation correcte ou incorrecte du module électronique, et donc en correspondance d'un flacon équipé dudit module électronique, lors des actionnements de l'actionneur. Dans un tel dispositif, l'équipement informatique exécutant le logiciel peut être adapté à émettre en temps réel un signal indiquant que le module a une orientation correcte pour la distribution de produit ophtalmique liquide par un flacon équipé dudit module électronique.

L'équipement informatique exécutant le logiciel peut dans certains modes de réalisation calculer sur le fondement des données transmises par le module un score d'observance et l'indiquer à l'utilisateur.

Cela permet ainsi à l'utilisateur de contrôler que sa gestuelle d'instillation est correcte. Si ce n'est pas le cas, l'utilisateur peut également la corriger, afin d'améliorer l'efficacité de son traitement. Les données temporelles d'instillation peuvent être combinées aux données caractérisant la gestuelle d'instillation pour calculer un « score » ou une « note », que l'utilisateur va naturellement chercher à améliorer pendant son traitement. Le dispositif a ainsi un effet pédagogique, voire ludique, pour l'utilisateur. L'équipement informatique peut par exemple être un téléphone intelligent ou une tablette, et le logiciel est alors une application.

D'autres particularités et avantages de l'invention apparaîtront encore dans la description ci-après.

Aux dessins annexés, donnés à titre d'exemples non limitatifs :
- la figure 1 représente, selon une vue schématique un flacon de produit ophtalmique liquide comportant une pompe mécanique, dans une première configuration ;
- la figure 2 représente, selon une vue schématique, le flacon de la figure 1 dans une deuxième configuration ;
- la figure 3 représente, selon une vue schématique, un flacon de produit ophtalmique liquide comportant un dispositif d'assistance à la délivrance ;
- la figure 4 représente, selon une vue schématique en trois dimensions, le flacon de la figure 3 ;
- la figure 5 représente, selon une vue schématique en trois dimensions, un module électronique conforme à un mode de réalisation de l'invention ;
- la figure 6 représente, selon une vue schématique en trois dimensions, le module de la figure 5 installé sur le flacon de la figure 3 ;
- la figure 7 représente, selon une vue tridimensionnelle de détail en coupe partielle, l'ensemble de la figure 6 ;
- la figure 8 représente, selon une vue schématique en trois dimensions, un autre module électronique installé sur un flacon analogue à celui des figures 3 et 4;
- la figure 9 représente, selon une vue schématique en trois dimensions, un module électronique selon un autre mode de réalisation de l'invention installé sur un flacon analogue à celui des figures 3 et 4 ;
- la figure 10 représente, selon une représentation schématique, un exemple d'un premier volet d'une interface d'application pouvant être mise en œuvre;
- la figure 11 représente, selon une représentation schématique, un exemple d'un deuxième volet d'une interface d'application pouvant être mise en œuvre;
- la figure 12 représente, selon une représentation schématique, un exemple d'un troisième volet d'une interface d'application pouvant être mise en œuvre;
- la figure 13 représente, selon une représentation schématique, un exemple d'un quatrième volet d'une interface d'application pouvant être mise en œuvre.

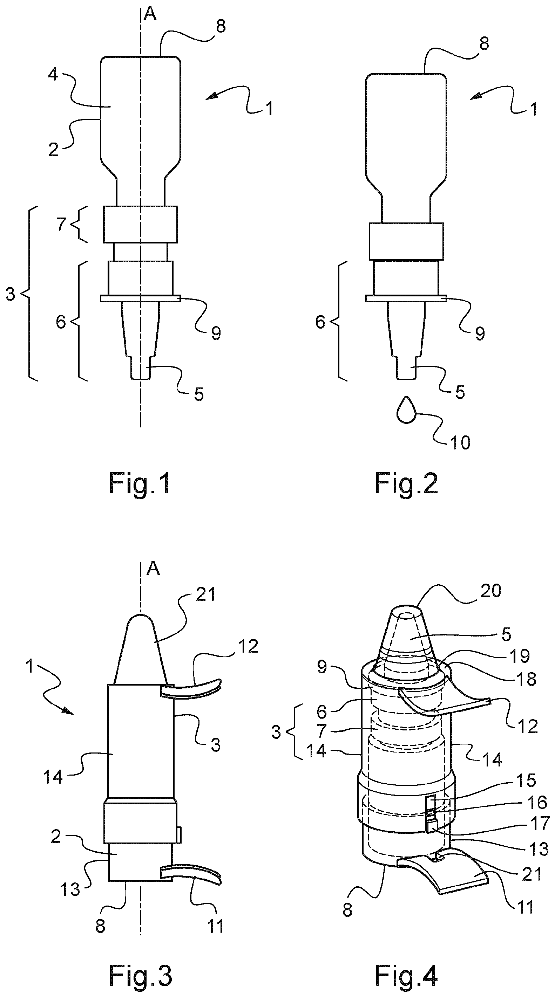

La figure 1 représente un flacon de produit ophtalmique liquide comportant une pompe mécanique. Un tel flacon, est connu dans l'état de la technique pour la distribution d'un produit liquide nasal ou oculaire. Le flacon 1 comporte un corps principal 2 et une tête de distribution 3. Le corps principal 2 forme un réservoir 4, destiné à contenir un produit liquide, par exemple un produit ophtalmique liquide. La tête de distribution 3 comporte un embout 5, doté à son extrémité d'un orifice de distribution par lequel le produit liquide sort lors de sa distribution. La tête de distribution 3 comporte une partie mobile 6 et une partie fixe 7. La partie 7 fixe étant rigidement liée au corps principal 2 du flacon 1, la partie mobile 6 est mobile vis-à-vis dudit corps principal 2. Plus particulièrement, la partie mobile 6 peut être translatée selon une direction dite axiale, déterminée par l'axe principal A d'extension du flacon 1. En particulier, le rapprochement de la partie mobile 6 d'un fond 8 du corps principal entraîne une pompe mécanique contenue dans le flacon. L'actionnement de la pompe mécanique entraine un prélèvement du liquide présent dans le réservoir 4 et sa distribution par l'embout de distribution 5. Afin d'aider l'utilisateur à actionner la pompe, c'est-à-dire à rapprocher la partie mobile 6 du fond 8, la partie mobile 6 comporte une collerette 9 formant un plan d'appui sensiblement orthogonal à la direction axiale d'actionnement.

La figure 1 représente le flacon 1 dans une configuration au repos, qu'il tend à adopter en l'absence d'actionnement.

La figure 2 représente le flacon 1 de la figure 1 dans une configuration d'actionnement, c'est-à-dire lorsque la partie mobile est actionnée par création d'une force tendant à la rapprocher du fond 8. Une telle force est par exemple obtenue par pincement entre les doigts d'une main de l'utilisateur, positionnés sur le fond 8 et sous la collerette 9. Lors de l'atteinte de la configuration d'actionnement de la figure 2, une goutte 10 ou un jet de produit est délivré par l'embout 5 du flacon 1.

Le flacon des figures 1 et 2 constitue un premier exemple de flacon sur lequel la présente invention peut être appliquée.

La figure 3 et la figure 4 représentent un flacon de produit ophtalmique liquide comportant un dispositif d'assistance à la délivrance. En effet, le flacon des figures 1 et 2 peut se révéler difficile à actionner, en particulier lors de la distribution d'un produit ophtalmique, une telle distribution nécessitant un maintien précis en position du flacon. Un bon maintien en position est obtenu en améliorant l'ergonomie générale du flacon et en facilitant l'application de la force nécessaire à son actionnement.

Le principe développé dans le flacon des figures 3 et 4 consiste à équiper le flacon 1 d'au moins deux ailettes latérales, sensiblement orthogonales à la direction axiale d'actionnement de la pompe et du flacon 1.

En particulier, le flacon 1 comporte une première ailette latérale 11 permettant le maintien en position de la partie fixe 7 du flacon 1 et une deuxième ailette latérale 12 permettant de bouger la partie mobile 6 du flacon 1 selon la direction axiale. Plus généralement, le rapprochement entre la première ailette latérale 11 et la deuxième ailette latérale 12 selon la direction axiale entraîne l'actionnement de la pompe mécanique du flacon 1.

La première ailette latérale 11 et la deuxième ailette latérale 12 sont sensiblement en regard l'une de l'autre, dans deux plans axialement distants. Cela facilite l'application d'une force tendant à les rapprocher. Dans l'exemple ici représenté, la première ailette latérale et la deuxième ailette latérales sont sensiblement de longueur identiques. D'autres configurations d'ailettes peuvent être employées, et sont compatibles de la présente invention.

En pratique, le flacon des figures 3 et 4 peut comporter le flacon des figures 1 et 2, sur lequel est ajouté une coiffe permettant de faciliter l'actionnement de la pompe. En particulier, le flacon des figures 1 et 2 est représenté en traits pointillés à la figure 4, installé dans une coiffe en deux parties formant un dispositif d'assistance à la délivrance.

Ainsi, le corps principal du flacon des figures 3 et 4 comporte une première partie du dispositif d'assistance à la délivrance 13 comportant la première ailette latérale 11, qui est rapportée au réservoir des figures 1 et 2. En particulier, la première partie du dispositif de délivrance 13 forme le fond 8 du flacon 1. La première partie du dispositif d'assistance à la délivrance 13 peut être configuré (hormis la première ailette latérale 11) essentiellement sous la forme d'une cuve dont la forme intérieure est adaptée à recevoir le réservoir 4.

La tête de distribution 3 du flacon des figures 3 et 4 comporte quant à elle, outre la partie fixe 7 et la partie mobile 6, une deuxième partie du dispositif d'assistance à la délivrance 14. La deuxième partie du dispositif d'assistance à la délivrance 14 comporte la deuxième ailette latérale 12. La deuxième partie du dispositif d'assistance à la délivrance 14 se présente (hormis la deuxième ailette latérale 12) essentiellement sous la forme d'un fut comportant une zone proximale du fond 8 coopérant avec la première partie du dispositif d'assistance à la délivrance 13 et un rebord distal 18 du fond 8 qui prend appui sur la collerette 9 de la partie mobile 6 de la tête de distribution. La première partie du dispositif d'assistance à la délivrance 13 et la deuxième partie du dispositif d'assistance à la délivrance 14 sont, dans l'exemple représenté, guidé en translation axiale l'un par rapport à l'autre à l'aide d'une rainure 15 de la deuxième partie du dispositif d'assistance à la délivrance 14 dans laquelle est guidée un pion 16 de la première partie du dispositif d'assistance à la délivrance 13. Une excroissance radiale 17 de la deuxième partie du dispositif d'assistance à la délivrance empêche le pion 16 de sortir de la rainure 15.

L'embout 5 du flacon 1 dépasse de l'extrémité ouverte distale 19 de la deuxième partie du dispositif d'assistance à la délivrance 14. Aux figures 3 et 4, le flacon 1 est représenté équipé d'un capuchon 20 qui obture et protège l'embout 5. D'autres configurations de capuchons sont bien évidemment connues, notamment comportant un indicateur visuel de première ouverture.

Le flacon des figures 3 et 4 constitue un deuxième exemple de flacon sur lequel la présente invention peut être appliquée, et en constitue une application préférentielle. Comme visible à la figure 4, le flacon 1 présente une adaptation optionnelle mineure (comparativement au flacon disponible commercialement dans l'art antérieur) permettant l'emploi sur ce flacon d'un module électronique formant un mode de réalisation préférentiel de l'invention. Cette adaptation consiste en la formation d'un trou 21 dans la première ailette latérale 11, à proximité immédiate de la paroi du corps principal 2 du flacon 1, dont l'emploi est expliqué ci-après.

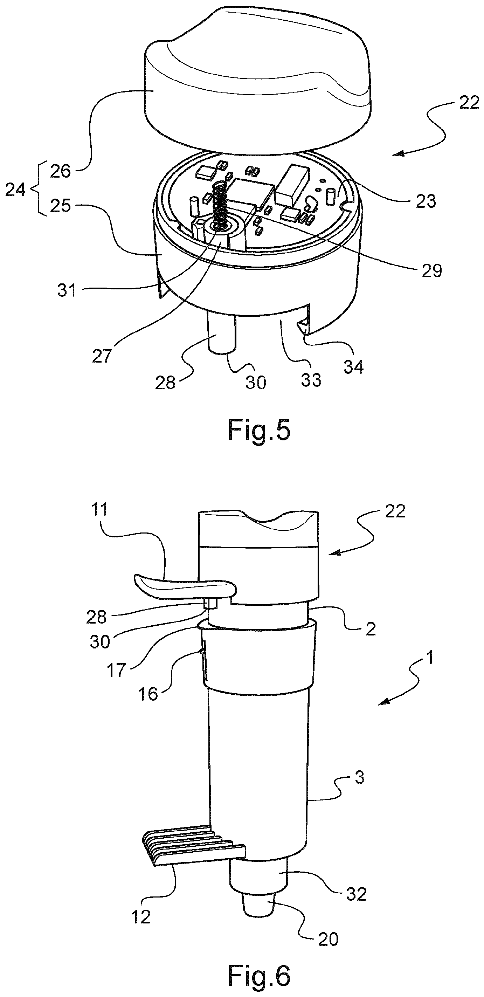

La figure 5 représente un module électronique 22 conforme à un mode de réalisation de l'invention. Le module électronique 22 comporte une ou plusieurs cartes électroniques 23, montées dans un boîtier 24. Le boîtier comporte, dans l'exemple représenté, une base 25 et un couvercle 26. Typiquement, la base 25 peut comporter des plots de réception et de maintien de la carte électronique 23. Une fois la carte électronique 23 positionnée dans la base, le boîtier est fermé par mise en place du couvercle 26. Le couvercle 26 est avantageusement (mais pas nécessairement) scellé sur la base 25, rendant impossible toute ouverture ultérieure. Des plots fusibles peuvent être utilisés pour réaliser ce scellement.

La base 25 comporte une ouverture 27 par laquelle un actionneur 28 sort du boîtier 24. La forme de l'ouverture correspondant à celle de l'actionneur 28, l'actionneur 28 est, dans l'exemple représenté, parfaitement guidé en translation dans l'ouverture 27. En l'occurrence, l'actionneur 28 se présente sous la forme d'une tige cylindrique. Un ressort 29, en l'occurrence un ressort hélicoïdal tend à ramener l'actionneur dans une position de repos, en l'absence de force extérieure appliquée sur l'actionneur 28.

Le module électronique 22 comporte un capteur destiné à être activé par le mouvement axial de l'actionneur 28. Avantageusement, le capteur est un contacteur. Ainsi l'actionneur 28 en forme de tige du mode de réalisation ici représenté présente à sa première extrémité, située hors du boîtier 24, une première surface d'appui 30. La première surface d'appui 30 est destinée à venir au contact et à prendre appui sur un élément de partie mobile 6 de la tête de distribution. L'actionneur 28 en forme de tige présente à sa deuxième extrémité, située dans le boîtier 24, une deuxième surface d'appui 31, destinée à vernir au contact et à appuyer sur le contacteur. Ainsi, la deuxième surface d'appui 31 provoque un appui ou un relâchement sur le contacteur lorsqu'une force est appliquée sur la première extrémité 30 de l'actionneur 28, que cette force est suffisante pour comprimer le ressort 29, et que le mouvement imposé à l'actionneur 28 a une amplitude suffisante.

Ainsi, dans la position de repos de l'actionneur 28, le contacteur est ouvert, tandis que lorsque l'actionneur est enfoncé dans le boîtier, le contacteur est fermé. La fermeture du contacteur peut, selon la technologie de contacteur considéré et la configuration du module électronique 22, être provoquée par un appui ou un relâchement sur le contacteur.

Le positionnement des composants électroniques du module dans un boîtier leur offre une protection adaptée contre les chocs et autres sollicitations mécaniques externes, mais aussi contre les particules et les éclaboussures.

Cela est particulièrement important pour un module qui est potentiellement destiné à être employé successivement sur plusieurs flacons. En particulier, l'actionnement indirect du capteur (par exemple du contacteur) à l'aide d'un actionneur qui est le seul élément traversant le boîtier du module, avec un très faible jeu fonctionnel, permet de protéger le capteur contre une détérioration de ses performances. Notamment, des particules ou poussières sur le capteur pourraient en fausser la mesure, et/ou créer des faux contacts.

Le module électronique 22 comporte une source d'énergie électrique, sous la forme d'une pile ou d'une batterie. Une pile peut assurer une autonomie très longue au capteur, de plusieurs semaines, plusieurs mois, voire plusieurs années de traitement. En particulier, lorsque le module électronique 22 comporte un contacteur en tant que capteur, le module électronique 22 peut être configuré pour ne consommer de l'énergie que lors de l'actionnement du capteur. Par exemple, la fermeture du capteur correspondant à la détection d'un actionnement de l'actionneur, et donc du flacon sur lequel il est monté, peut entraîner l'alimentation du module électronique 22.

La figure 6 représente le module de la figure 5 installé sur le flacon analogue à celui des figures 3 et 4. Le flacon représenté à la figure 6 diffère uniquement de celui des figures 3 et 4 en ce que le capuchon 20 est de forme différente et présente une bague 32 permettant de visualiser si le flacon a déjà été ouvert ou non.

Le module électronique 22 est fixé, dans l'exemple ici représenté, sur le fond 8 du flacon 1, par correspondance de forme et par clipsage sur la première ailette latérale 11. Le clipsage du module électronique 22 sur la première partie du dispositif d'assistance à la délivrance 13 rend ces deux éléments solidaires, et fixes l'un par rapport à l'autre. Pour cela, la base 25 du boîtier 24 est pourvue d'une échancrure 33 formant deux pattes de fixation 34 adaptée à venir se bloquer sous la première ailette latérale 11.

L'actionneur 28 traverse le trou formé dans la première ailette latérale 11. Lorsque le flacon est actionné par rapprochement de la première ailette latérale 11 et de la deuxième ailette latérale 12 selon la direction axiale (parallèle à l'axe principal A du flacon), la première surface d'appui 30 de l'actionneur vient au contact de l'excroissance radiale 17 de la deuxième partie du dispositif d'assistance à la délivrance 14. Le mouvement du module électronique vis-à-vis de l'excroissance radiale 17 se poursuivant, celle-ci applique par réaction une force sur l'actionneur 28 qui pousse l'actionneur dans le boîtier 24. Le contacteur du module électronique 22 est alors actionné. Le trou dans l'ailette latérale 11 garantit un positionnement correct de l'actionneur et du module vis-à-vis du flacon. En outre, il participe au bon guidage en translation de l'actionneur.

Bien évidemment une fixation du module au corps principal du flacon, en une autre localisation que son fond et/ou par un autre moyen que le clipsage est envisageable sans sortir du cadre de l'invention.

Le module électronique peut notamment être configuré de sorte que le boîtier qu'il comporte soit installé sur le côté, et non à une extrémité, du flacon équipé. Cela limite l'augmentation de longueur du flacon liée à l'installation du module électronique.

L'actionnement du contacteur (ou autre capteur) du module électronique 22 provoque soit l'enregistrement de données relatives à cet actionnement, soit la transmission sans fil de ces données, soit les deux. L'enregistrement des données peut être réalisé sur une mémoire que comporte le cas échéant le module électronique 22.

La transmission des données, en temps réel ou ponctuellement après enregistrement, est avantageusement réalisée sans fil, par exemple selon un protocole Bluetooth^{™}, avantageusement Bluetooth Low Energy ^{™}. A cette fin, le module électronique 22 est doté d'un système de communication sans fil, comportant un port de communication sans fil assurant le transfert des données. Dans un mode de réalisation alternatif, une communication via un port filaire peut être employée.

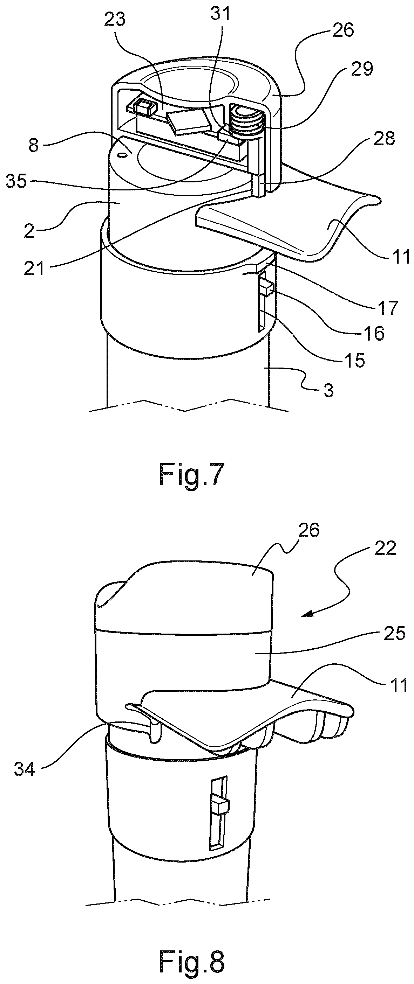

Dans le mode de réalisation de la figure 7, un contacteur 35 est sous la pression de la deuxième surface d'appui 31 de l'actionneur 28, sous l'effet du ressort 29, lorsque l'actionneur est dans sa position de repos. La mise en mouvement de l'actionneur 28 lors de son appui, via la première surface d'appui 30, sur l'excroissance radiale 17 de la deuxième partie du dispositif d'assistance à la délivrance 14, relâche la pression exercée sur le contacteur 35 par la deuxième surface d'appui 31, ce qui provoque la fermeture du contacteur, et la détection de l'actionnement du flacon.

Les données relatives à l'actionnement peuvent être diverses. La première des données porte sur l'information même d'actionnement du flacon. A cette information peut être associée une information temporelle, typiquement une information de date et d'heure de l'actionnement détectée, ou une information relative au temps écoulé depuis le précédent actionnement. L'information d'actionnement peut être cumulée au cours du temps, afin de déterminer un nombre d'actionnements lors d'une prise (lors d'une séquence d'instillation), ou un nombre d'actionnements total.

Le module peut comporter un ou plusieurs accéléromètres. En alternative ou complément, d'autres capteurs de position et d'orientation, notamment des gyroscopes, peuvent être utilisés. L'accéléromètre permet de déterminer l'orientation du module électronique dans l'espace, et en particulier son orientation par rapport à la verticale. Pour que l'instillation soit correcte, il est nécessaire que le flacon soit orienté verticalement, c'est-à-dire que l'axe principal A du flacon soit aligné avec la verticale, ou à tout le moins compris dans une plage d'angles donnée autour de la verticale. Cette information est déterminée à l'aide de l'accéléromètre au moment de l'actionnement du flacon, et peut être associée aux données relatives à l'actionnement, notamment à une information temporelle.

Le module électronique peut ainsi permettre, dans certains modes de réalisation, de déterminer quand une instillation a été réalisée, combien de gouttes de produit ont été délivrées à cette occasion, et quelle était l'orientation du flacon pour chaque goutte délivrée.

La figure 8 représente un autre module électronique installé sur le flacon des figures 3 et 4. Ce mode de réalisation diffère de celui des figures 5 à 7 en ce que la base 25 du boîtier 24 du module électronique 22 comporte des pattes 34 de clipsage sur la première ailette latérale 11 qui sont de plus grandes dimensions et qui s'étendent en partie latéralement, sous ladite première ailette latérale 11. La forme bombée, optionnelle, de la première ailette latérale 11 permet un clipsage aisé par pression axiale sur le module 22, cette pression et ladite forme bombée ayant pour effet d'écarter les pattes 34. Le retrait du module électronique 22, par exemple afin de l'installer sur un autre flacon, est réalisé en écartant les pattes 34 l'une de l'autre, ce qui dégage les pattes de la première ailette latérale 11. La fixation du module électronique 22 est ainsi fiabilisé, et les jeux entre le module électronique 22 et le flacon sont limités.

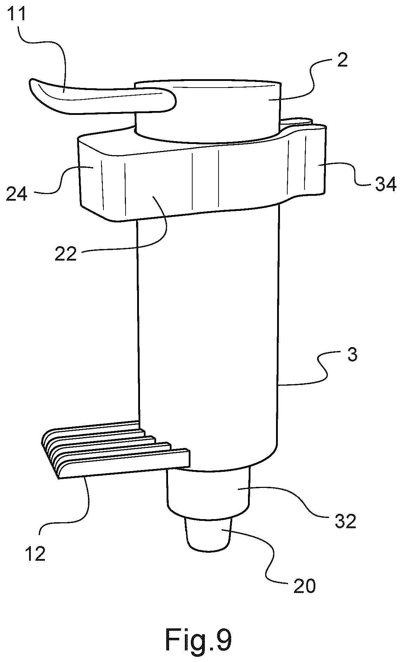

L'invention a été illustrée aux figures 5 à 8 selon des modes de réalisation dans lesquels le module électronique 22 est fixé au corps principal 2 du flacon. De manière analogue, une fixation du module électronique 22 à la tête de distribution 3 est possible, dans une configuration du module électronique 22 selon laquelle le mouvement relatif entre le corps principal 2 et la partie mobile 6 permet l'actionnement dudit module électronique 22. Un tel mode de réalisation est illustré à la figure 9.

Plus précisément, dans le mode de réalisation représenté à la figure 9, le module électronique 22 est clipsé sur la tête de distribution sur la deuxième partie du dispositif d'assistance à la délivrance 14 qui coopère avec la partie mobile 6. A cette fin, le module électronique 22 est doté de deux larges pattes de fixation 34 qui enserrent la partie extrême de la deuxième partie du système de délivrance 14. Le boîtier 24 du module électronique 22 recouvre, dans l'exemple ici représenté la rainure 15 et le pion 16 de la première partie du système de délivrance 13 qui est guidé dans la rainure 15. Le pion 16 peut être utilisé pour actionner l'actionneur du module électronique (non visible sur la figure 9, car situé sur la face du boîtier 24 qui est au contact du flacon 1). Le mouvement du pion 16 dans la rainure 15, qui traduit le mouvement de la partie mobile 6 vis-à-vis du corps principal 2, entraine le mouvement de l'actionneur du module 22 et traduit une instillation de produit par le flacon.

La transmission, en temps réel ou non, des données issues du module électronique permet de contrôler les paramètres d'instillation du produit et l'observance du traitement. La transmission est réalisée à destination d'un équipement informatique, par exemple un ordinateur, un serveur, une tablette ou un téléphone intelligent (généralement désigné par le terme anglophone « smartphone »). L'équipement informatique exécute un logiciel permettant la récupération, le traitement, et l'affichage des données (brutes ou après traitement) issues du module électronique 22 (ou de plusieurs modules).

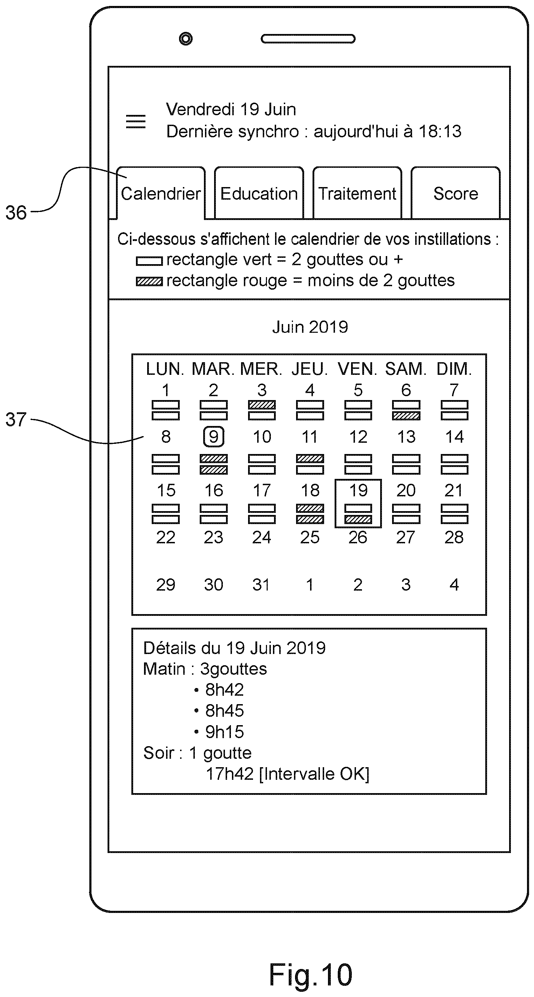

Les figures 10 à 13 représentent divers volets d'une application exécutée sur un smartphone connecté au module électronique 22. Ces différents volets illustrent, à titre d'exemples, différentes fonctionnalités pouvant être offertes par le module électronique 22 et l'application exécutée.

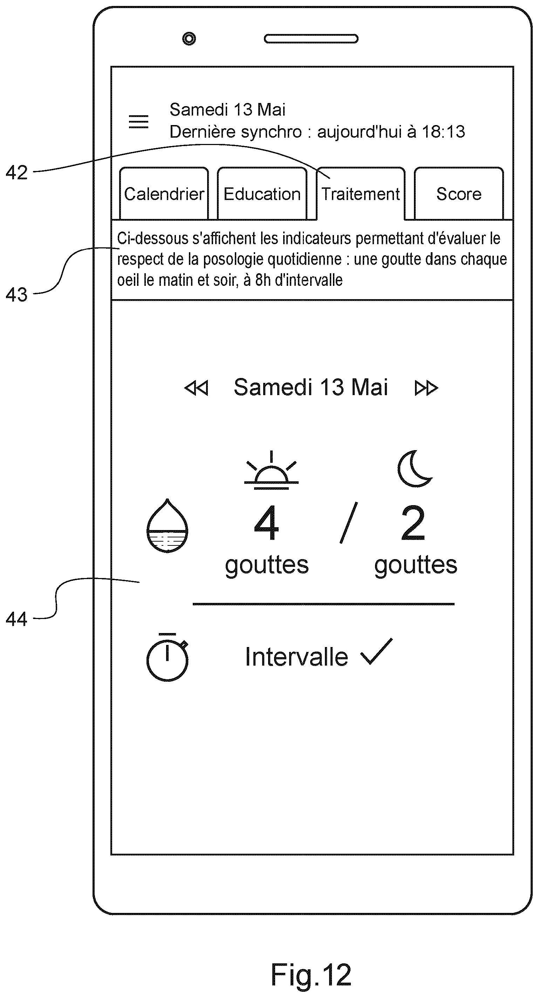

La figure 10 représente un volet « calendrier » 36 dédié au contrôle de l'observance dans le temps d'un traitement. Selon la posologie indiquée par ailleurs dans l'application (voir figure 12 décrite ci-après), ce volet calendrier 36 offre une vue générale temporelle de l'observance du traitement. Le volet calendrier peut notamment proposer une vue mensuelle 37 (ou alternativement, hebdomadaire, sur deux semaines, etc.). Pour chaque jour est représenté un rectangle correspondant à une instillation prévue (par exemple, pour une installation le matin et une instillation le soir, deux rectangles sont représentés pour chaque jour). Pour les instillations correctement réalisées, un indicateur visuel, ici sous la forme d'un rectangle blanc est affiché. Bien sur d'autres indicateurs, et ou d'autres couleurs peuvent être employés. Notamment, un indicateur vert est spontanément associé à une action positive, et peut donc avantageusement représenter une instillation correcte. Par instillation correcte, on entend la détection d'un actionnement du flacon dans une plage temporelle donnée, ou le cas échéant plusieurs actionnements du flacon correspondant au nombre de gouttes à délivrer, et/ou la détection dans la plage temporelle donnée d'une orientation adéquate du flacon.

A contrario, une instillation incorrecte est représentée par un indicateur différent, ici un rectangle hachuré. Bien sur d'autres indicateurs, et ou d'autres couleurs peuvent être employés. Notamment, un indicateur rouge est spontanément associé à une action négative, et peut donc avantageusement représenter une instillation incorrecte.

Une action (clic, pointage, pression) sur un jour de la vue mensuelle 37 permet l'affichage de détails sur les instillations de ce jour, dans une zone de détail 38. La zone de détail peut par exemple fournir l'heure exacte de délivrance de chaque goutte, d'autres informations tel que le respect correct ou non de l'intervalle prévu dans la posologie de traitement, ou encore diverses informations liées à des erreurs possibles dans le système, la mesure, etc.

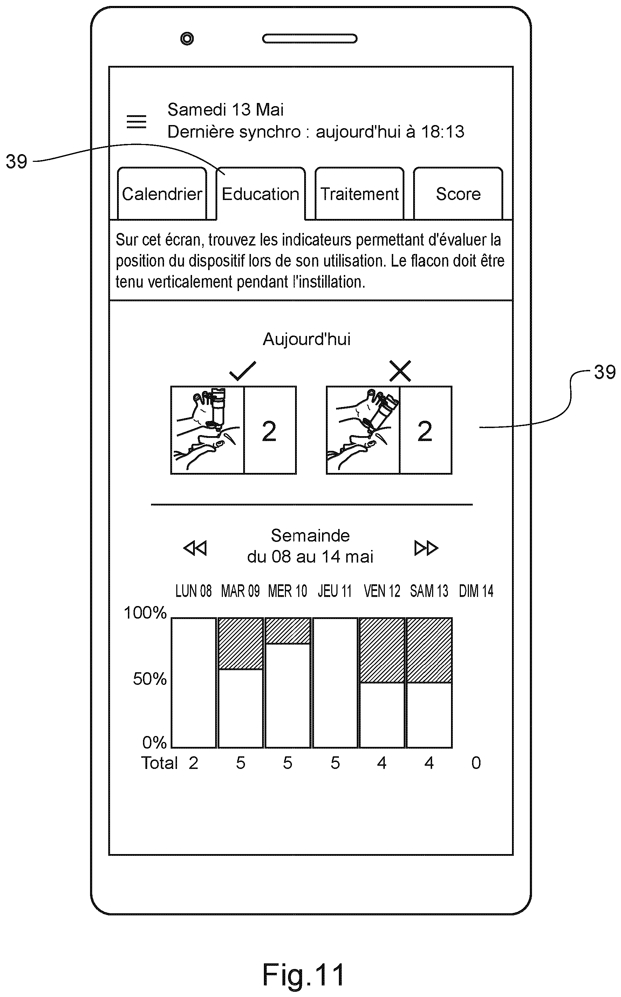

La figure 11 représente un volet « éducation » 39, dédié au contrôle et à l'apprentissage par l'utilisateur de la gestuelle correcte d'instillation. Ce volet permet d'indiquer, dans certains modes de réalisation, si une installation a été réalisée avec le flacon correctement orienté, c'est-à-dire avec son axe principal A correctement aligné avec la verticale. Le résultat peut être affiché en temps réel, immédiatement après l'actionnement du flacon, ce qui permet à l'utilisateur de corriger la position du flacon pour l'actionnement suivant du flacon.

Différentes vues concernant les instillations passées peuvent être disponibles dans ce volet. Dans l'exemple ici représenté, une vue quotidienne 40 indique le nombre d'instillations réalisées avec une orientation correcte et le nombre d'instillations réalisées avec une orientation incorrecte dans la journée courante. Dans l'exemple représenté, une vue hebdomadaire 41 apporte une information sur la proportion d'instillations réalisées avec une orientation correcte et la proportion d'instillations réalisées avec une orientation sur la semaine courante, ou sur une semaine glissante, ou plus généralement sur plusieurs jours. La répartition est par exemple représentée pour chaque jour par un histogramme, les instillations réalisées avec une orientation correcte étant représentées par une barre blanche, et les instillations réalisées avec une orientation incorrecte étant représentées par une barre hachurée. D'autres indicateurs et/ou d'autres couleurs (par exemple vert et rouge) peuvent être utilisés. D'autres catégories peuvent être illustrées, par exemple les instillations réalisées avec une orientation à la limite ou une orientation incertaine (par exemple représentées par un indicateur orange).

L'un des objectifs de ce volet éducation 39 est de permettre à l'utilisateur de vérifier si l'orientation donnée au flacon lors de l'instillation de gouttes ophtalmiques est acceptable, et de corriger cette orientation si besoin tout au long de son traitement. La figure 12 représente un volet « traitement » 42, dédié à la visualisation et le cas échéant à la détermination de la posologie. En particulier, ce volet peut servir à programmer la posologie du traitement, qui sera utilisée par les autres volets Ce volet présente également un rappel de la posologie 43. Il présente également une zone de contrôle de la posologie, qui indique le nombre de gouttes distribuées comparativement à la posologie souhaitée, et qui indique si l'intervalle entre deux instillations est respecté (c'est-à-dire situé dans une plage temporelle admissible). Le contrôle de l'intervalle est important, par exemple lorsque l'utilisateur voyage dans différents fuseaux horaires, de sorte que le seul suivi du traitement sur la base de l'heure locale n'est pas suffisant.

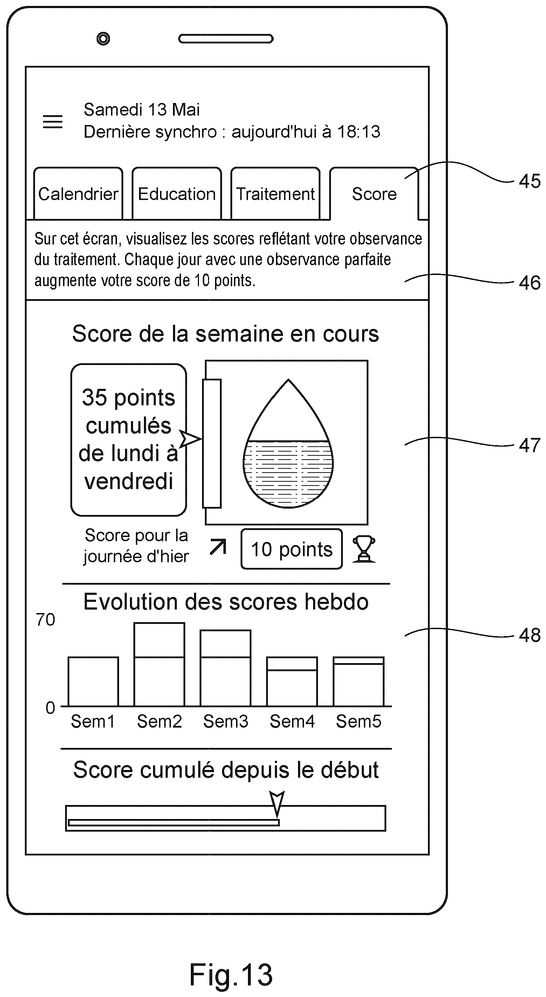

La figure 13 représente un volet « score » 45. Le volet score 45 traite et consolide les données reçues du module électronique 22 pour calculer un score, sous forme de points, de couleur, de progression sur une échelle, etc., qui traduit la bonne observance du traitement. Cette bonne observance consiste au respect de la posologie prescrite, mais aussi à l'adoption d'un geste d'instillation correct (traduit par la bonne orientation du flacon lors de la distribution du produit ophtalmique). C'est avant tout une façon ludique d'inciter l'utilisateur à suivre au mieux son traitement, en l'incitant, consciemment ou non, à améliorer son score. Le volet score peut comporter un rappel de la règle de calcul employée 46. Le volet score comporte une zone d'affichage du score hebdomadaire 47 (semaine en cours ou semaine glissante).

Le score de chaque semaine peut être mémorisé, de sorte à présenter l'évolution des scores hebdomadaires dans une zone d'évolution des scores 48. Enfin, une zone de score cumulé 49 présente un score fondé sur l'ensemble du traitement, par exemple sous la forme d'une barre de progression ou d'un curseur indiquant un score d'observance moyen.

Le module ainsi développé dans l'invention permet la détection des actionnements d'un flacon à pompe mécanique, en particulier à actionnement axial (selon un axe principal du flacon). La détection des actionnements, couplée à des informations temporelles (instant d'actionnement, intervalles entre les actionnements) ainsi que, le cas échéant, à des informations concernant l'orientation du flacon lors de l'actionnement de sa pompe, permet de contrôler l'observance et la qualité d'instillation d'un traitement, selon la posologie prescrite. Le fait que le module soit indépendant du flacon offre de multiples avantages. Par exemple, le module peut être transféré sur plusieurs flacons au cours d'un traitement de longue durée. Le module peut être prescrit à certains patients seulement : patients susceptibles d'oublier de suivre leur traitement, patients susceptibles de douter après quelques instants de s'être instillé ou non le produit ophtalmique, patients débutant un traitement. En outre, il est possible d'équiper un flacon préexistant, c'est-à-dire déjà sur le marché, uniquement lorsque cela est souhaité ou nécessaire. Ainsi, sans reconstruire une gamme de produits ophtalmiques, il est possible de proposer un même flacon sans module ou avec un module indépendant, selon le produit que contient le flacon, selon le marché (zone géographique), etc.

Le module peut également permettre d'apprendre à l'utilisateur une gestuelle d'instillation correcte, nécessaire à une bonne efficacité du traitement. Lorsque le module électronique est lié, par exemple apparié, avec un équipement informatique exécutant un logiciel approprié, par exemple un téléphone intelligent exécutant une application dédiée, les données obtenues par le module électronique et transférée à l'équipement électronique peuvent être traitées pour caractériser l'observance du traitement, et, le cas échéant, inciter l'utilisateur à l'améliorer.

## Revendications

1. Module électronique (22) destiné à détecter et à transmettre et/ou enregistrer des données relatives à l'actionnement d'une pompe mécanique d'un flacon (1) d'un produit ophtalmique liquide comportant un corps principal (2) comportant un réservoir (4), une tête de distribution (3) et une pompe mécanique, la tête de distribution (3) étant montée sur le corps principal, la tête de distribution (3) comportant une partie mobile (6) dont un mouvement relativement au corps principal (2) actionne la pompe mécanique pour prélever le produit ophtalmique liquide présent dans le réservoir (4), **caractérisé en ce que** ledit module électronique (22) est indépendant dudit flacon (1) de sorte qu'il peut y être rapporté et fixé, ledit module électronique (22) comportant un actionneur (28) configuré pour être entraîné en mouvement par le mouvement de la partie mobile (6), ledit module comportant un capteur configuré de sorte à être actionné par le mouvement de l'actionneur (28),
et **en ce que** le module électronique (22) comporte un boîtier (24) formant un volume interne de réception d'au moins une carte électronique (23), ledit boîtier comportant une base et un couvercle, ladite base comportant une ouverture permettant le passage de l'actionneur (28).

2. Module électronique (22) selon la revendication 1, adapté à un flacon dans lequel la partie mobile (6) est mobile en translation axiale relativement au corps principal (2), dans lequel le capteur est un contacteur (35), l'actionneur (28) est guidé en translation selon ladite direction axiale, et l'actionneur (28) comporte une première surface d'appui (30) sur la partie mobile (6) et une deuxième surface d'appui (31) sur le contacteur (35).

3. Module électronique (22) selon la revendication 2, dans lequel l'actionneur (28) se présente sous la forme d'une tige s'étendant selon la direction axiale, la tige comportant une première extrémité formant la première surface d'appui (30), et une deuxième extrémité, opposée à la première extrémité, formant la deuxième surface d'appui (31).

4. Module électronique (22) selon l'une des revendications précédentes, comportant un ressort (29) tendant à ramener l'actionneur (28) dans une position de repos.

5. Module électronique (22) selon les revendications 2 et 4 dans lequel le contacteur (35) est ouvert lorsque l'actionneur (28) est dans la position de repos.

6. Module électronique (22) selon l'une des revendications précédentes, comportant au moins un accéléromètre configuré pour fournir une information d'orientation du module électronique (22).

7. Module électronique (22) selon l'une des revendications précédentes, comportant une mémoire électronique configurée pour stocker des données relatives aux actionnements de l'actionneur (28), notamment le nombre, la date et l'heure des actionnements.

8. Module électronique (22) selon l'une des revendications précédentes, comportant un port de communication, filaire ou sans fil, configuré pour le transfert de données vers un équipement informatique externe.

9. Module électronique (22) selon la revendication 8, dans lequel le module est configuré pour transférer les données vers l'équipement informatique externe selon un protocole Bluetooth^{™}.

10. Ensemble comportant un flacon (1) d'un produit ophtalmique liquide et un module électronique (22) selon l'une des revendications précédentes,
le flacon (1) comportant un corps principal (2) comportant un réservoir (4), une tête de distribution (3), et une pompe mécanique, la tête de distribution (3) étant montée sur le corps principal (2), la tête de distribution (3) comportant une partie mobile (6) dont un mouvement actionne la pompe mécanique pour prélever le produit ophtalmique liquide présent dans le réservoir (4),
le module électronique (22) étant rapporté et fixé soit au corps principal soit à la tête de distribution (3) de sorte que le mouvement de la partie mobile (6) relativement au corps principal (2) pour prélever le produit ophtalmique liquide présent dans le réservoir (4) entraine en mouvement l'actionneur (28) du module électronique (22).

11. Ensemble selon la revendication 10, dans lequel le flacon (1) comporte un dispositif d'assistance à la délivrance et dans lequel la partie mobile (6) est mobile en translation axiale relativement au corps principal (2),
le corps principal (2) comportant une première partie du dispositif d'assistance à la délivrance (13) qui coopère avec le réservoir (4) dudit corps principal (2), la première partie du dispositif d'assistance à la délivrance (13) comportant une première ailette latérale (11),
la tête de distribution (3) comportant une deuxième partie du dispositif d'assistance à la délivrance (14) qui coopère avec la partie mobile (6), la deuxième partie du dispositif d'assistance à la délivrance (14) comportant une deuxième ailette latérale (12),
la première ailette latérale (11) et la deuxième ailette latérale (12) étant sensiblement en regard l'une de l'autre pour permettre le mouvement axial de la partie mobile (6) lorsqu'une force est exercée entre les deux ailettes latérales (11,12) tendant à les rapprocher axialement l'une de l'autre.

12. Ensemble selon la revendication 11, dans lequel, lors du mouvement axial de la partie mobile (6), l'actionneur (28) prend appui sur, et est actionné par, la deuxième partie du dispositif d'assistance à la délivrance (14).

13. Ensemble selon la revendication 12, dans lequel la première ailette latérale (11) comporte un trou traversé par l'actionneur (28) du module électronique (22), ledit actionneur (28) prenant appui lors du mouvement axial de la partie mobile (6) sur une excroissance radiale (17) de la deuxième partie du dispositif d'assistance à la délivrance (14).

## Patentansprüche

1. Elektronisches Modul (22), das zum Erkennen und Übertragen und/oder Speichern von Daten in Bezug auf die Betätigung einer mechanischen Pumpe eines Fläschchens (1) eines flüssigen ophthalmologischen Produkts bestimmt ist, das einen Hauptkörper (2) umfasst, der einen Behälter (4), einen Ausgabekopf (3) und eine mechanische Pumpe umfasst, wobei der Ausgabekopf (3) am Hauptkörper montiert ist, wobei der Ausgabekopf (3) einen beweglichen Teil (6) umfasst, dessen Bewegung relativ zum Hauptkörper (2) die mechanische Pumpe betätigt, um das in dem Behälter (4) vorhandene flüssige ophthalmologische Produkt zu entnehmen, **dadurch gekennzeichnet, dass** das elektronische Modul (22) von dem Fläschchen (1) unabhängig ist, so dass es an diesem angebracht und befestigt werden kann, wobei das elektronische Modul (22) eine Betätigungsvorrichtung (28) umfasst, die so konfiguriert ist, dass sie durch die Bewegung des beweglichen Teils (6) in Bewegung versetzt wird, wobei das Modul einen Sensor umfasst, der so konfiguriert ist, dass er durch die Bewegung der Betätigungsvorrichtung (28) betätigt wird,
und dadurch, dass das elektronische Modul (22) ein Gehäuse (24) umfasst, das ein inneres Volumen zum Aufnehmen mindestens einer elektronischen Karte (23) bildet, wobei das Gehäuse eine Basis und einen Deckel umfasst, wobei die Basis eine Öffnung umfasst, die den Durchtritt der Betätigungsvorrichtung (28) ermöglicht.

2. Elektronisches Modul (22) nach Anspruch 1, das für ein Fläschchen geeignet ist, bei dem der bewegliche Teil (6) relativ zum Hauptkörper (2) axial verschiebbar ist, wobei der Sensor ein Schalter (35) ist, die Betätigungsvorrichtung (28) in der axialen Richtung verschiebegeführt ist, und die Betätigungsvorrichtung (28) eine erste Auflagefläche (30) am beweglichen Teil (6) und eine zweite Auflagefläche (31) am Schalter (35) umfasst.

3. Elektronisches Modul (22) nach Anspruch 2, wobei sich die Betätigungsvorrichtung (28) in Form eines Stabes zeigt, der sich in der axialen Richtung erstreckt, wobei der Stab ein erstes Ende umfasst, das die erste Auflagefläche (30) bildet, und ein dem ersten Ende gegenüberliegendes zweites Ende, das die zweite Auflagefläche (31) bildet.

4. Elektronisches Modul (22) nach einem der vorstehenden Ansprüche, das eine Feder (29) umfasst, die dazu dient, die Betätigungsvorrichtung (28) in eine Ruheposition zurückzuführen.

5. Elektronisches Modul (22) nach den Ansprüchen 2 und 4, wobei der Schalter (35) geöffnet ist, wenn sich die Betätigungsvorrichtung (28) in der Ruheposition befindet.

6. Elektronisches Modul (22) nach einem der vorstehenden Ansprüche, das mindestens einen Beschleunigungsmesser umfasst, der so konfiguriert ist, dass er eine Information über die Ausrichtung des elektronischen Moduls (22) bereitstellt.

7. Elektronisches Modul (22) nach einem der vorstehenden Ansprüche, das einen elektronischen Speicher umfasst, der so konfiguriert ist, dass er Daten in Bezug auf die Betätigungen der Betätigungsvorrichtung (28), insbesondere die Anzahl, das Datum und die Uhrzeit der Betätigungen, speichert.

8. Elektronisches Modul (22) nach einem der vorstehenden Ansprüche, das einen drahtgebundenen oder drahtlosen Kommunikationsanschluss umfasst, der für die Übertragung von Daten an eine externe IT-Ausrüstung konfiguriert ist.

9. Elektronisches Modul (22) nach Anspruch 8, wobei das Modul so konfiguriert ist, dass es die Daten gemäß einem Bluetooth^{™}-Protokoll an die externe IT-Ausrüstung überträgt.

10. Anordnung, die ein Fläschchen (1) für ein flüssiges ophthalmologisches Produkt und ein elektronisches Modul (22) nach einem der vorstehenden Ansprüche umfasst,
wobei das Fläschchen (1) einen Hauptkörper (2), der einen Behälter (4) umfasst, einen Ausgabekopf (3) und eine mechanische Pumpe umfasst, wobei der Ausgabekopf (3) am Hauptkörper (2) montiert ist, wobei der Ausgabekopf (3) einen beweglichen Teil (6) umfasst, dessen Bewegung die mechanische Pumpe betätigt, um das in dem Behälter (4) vorhandene flüssige ophthalmologische Produkt zu entnehmen,
wobei das elektronische Modul (22) entweder am Hauptkörper oder am Ausgabekopf (3) angebracht und befestigt ist, sodass die Bewegung des beweglichen Teils (6) relativ zum Hauptkörper (2), um das in dem Behälter (4) vorhandene flüssige ophthalmologische Produkt zu entnehmen, die Betätigungsvorrichtung (28) des elektronischen Moduls (22) in Bewegung versetzt.

11. Anordnung nach Anspruch 10, wobei das Fläschchen (1) eine Abgabehilfsvorrichtung umfasst und wobei der bewegliche Teil (6) relativ zum Hauptkörper (2) axial verschiebbar ist,
wobei der Hauptkörper (2) einen ersten Teil der Abgabehilfsvorrichtung (13) umfasst, der mit dem Behälter (4) des Hauptkörpers (2) zusammenwirkt, wobei der erste Teil der Abgabehilfsvorrichtung (13) einen ersten seitlichen Flügel (11) umfasst,
wobei der Ausgabekopf (3) einen zweiten Teil der Abgabehilfsvorrichtung (14) umfasst, der mit dem beweglichen Teil (6) zusammenwirkt, wobei der zweite Teil der Abgabehilfsvorrichtung (14) einen zweiten seitlichen Flügel (12) umfasst,
wobei der erste seitliche Flügel (11) und der zweite seitliche Flügel (12) einander im Wesentlichen zugewandt sind, um die axiale Bewegung des beweglichen Teils (6) zu ermöglichen, wenn eine Kraft zwischen den zwei seitlichen Flügeln (11, 12) ausgeübt wird, die dazu dient, diese axial einander anzunähern.

12. Anordnung nach Anspruch 11, wobei bei der axialen Bewegung des beweglichen Teils (6) die Betätigungsvorrichtung (28) am zweiten Teil der Abgabehilfsvorrichtung (14) in Auflage geht und von diesem betätigt wird.

13. Anordnung nach Anspruch 12, wobei der erste seitliche Flügel (11) ein Loch umfasst, durch das die Betätigungsvorrichtung (28) des elektronischen Moduls (22) hindurchtritt, wobei die Betätigungsvorrichtung (28) bei der axialen Bewegung des beweglichen Teils (6) an einem radialen Wulst (17) des zweiten Teils der Abgabehilfsvorrichtung (14) in Auflage geht.

## Claims

1. An electronic module (22) for detecting and transmitting and/or recording data relating to the actuation of a mechanical pump of a vial (1) of a liquid ophthalmic product comprising a main body (2) comprising a reservoir (4), a dispensing head (3) and a mechanical pump, the dispensing head (3) being mounted to the main body, the dispensing head (3) comprising a movable part (6) a movement of which relative to the main body (2) actuates the mechanical pump to draw the liquid ophthalmic product present in the reservoir (4), **characterised in that** said electronic module (22) is independent of said vial (1) so that it can be assembled and fastened thereto, said electronic module (22) comprising an actuator (28) configured to be movably driven by the movement of the movable part (6), said module comprising a sensor configured to be actuated by the movement of the actuator (28),
and **in that** the electronic module (22) comprises a casing (24) forming an internal volume for receiving at least one electronic board (23), said casing comprising a base and a lid, said base comprising an opening for the actuator (28) to pass therethrough.

2. The electronic module (22) according to claim 1, adapted to a vial in which the movable part (6) is axially translationally movable relative to the main body (2), wherein the sensor is a contactor (35), the actuator (28) is translationally guided along said axial direction, and the actuator (28) comprises a first bearing surface (30) bearing on the movable part (6) and a second bearing surface (31) bearing on the contactor (35).

3. The electronic module (22) according to claim 2, wherein the actuator (28) is in the form of a rod extending along the axial direction, the rod comprising a first end forming the first bearing surface (30), and a second end, opposite to the first end, forming the second bearing surface (31) .

4. The electronic module (22) according to one of the preceding claims, comprising a spring (29) aiming to return the actuator (28) to a rest position.

5. The electronic module (22) according to claims 2 and 4, wherein the contactor (35) is open when the actuator (28) is in the rest position.

6. The electronic module (22) according to one of the preceding claims, comprising at least one accelerometer configured to provide orientation information of the electronic module (22).

7. The electronic module (22) according to one of the preceding claims, comprising an electronic memory configured to store data relating to actuations of the actuator (28), especially the number, date and time of actuations.

8. The electronic module (22) according to one of the preceding claims, comprising a wired or wireless communication port, configured to transfer data to external computing equipment.

9. The electronic module (22) according to claim 8, wherein the module is configured to transfer data to the external computing equipment according to a Bluetooth^{™} protocol.

10. An assembly comprising a vial (1) of a liquid ophthalmic product and an electronic module (22) according to one of the preceding claims,
the vial (1) comprising a main body (2) comprising a reservoir (4), a dispensing head (3), and a mechanical pump, the dispensing head (3) being mounted to the main body (2), the dispensing head (3) comprising a movable part (6) a movement of which actuates the mechanical pump to draw the liquid ophthalmic product present in the reservoir (4),
the electronic module (22) being assembled and fastened either to the main body or to the dispensing head (3) such that the movement of the movable part (6) relative to the main body (2) to draw the liquid ophthalmic product present in the reservoir (4) movably drives the actuator (28) of the electronic module (22).

11. The assembly according to claim 10, wherein the vial (1) comprises a delivery assist device and wherein the movable part (6) is axially translationally movable relative to the main body (2),
the main body (2) comprising a first part of the delivery assist device (13) that cooperates with the reservoir (4) of said main body (2), the first part of the delivery assist device (13) comprising a first side fin (11),
the dispensing head (3) comprising a second part of the delivery assist device (14) that cooperates with the movable part (6), the second part of the delivery assist device (14) comprising a second side fin (12),
the first side fin (11) and the second side fin (12) being substantially facing each other to allow axial movement of the movable part (6) when a force is exerted between the two side fins (11, 12) aiming to axially bring them closer to each other.

12. The assembly according to claim 11, wherein, during the axial movement of the movable part (6), the actuator (28) bears on, and is actuated by, the second part of the delivery assist device (14).

13. The assembly according to claim 12, wherein the first side fin (11) comprises a hole through which the actuator (28) of the electronic module (22) passes, said actuator (28) bearing on a radial protrusion (17) of the second part of the delivery assist device (14) during the axial movement of the movable part (6).
